# EUROPEAN PATENT APPLICATION

(11) **EP 2 703 555 A1**
(43) Date of publication of application: **05.03.2014**
(21) Application number: 13464004.4
(22) Date of filing: 02.04.2013
(51) Int. Cl.: D06M 11/36, A61L 9/16, D06M 11/47, D06M 11/48, D06M 11/68, D06M 16/00, A62D 3/30

(54) **Functionalization process of cellulosic textile materials (fibres, yarns, fabrics and knitted fabrics) such as:cotton, hemp, flax, ramie, nettle, etc., and different mixtures of such fibres**

(30) Priority: 04.09.2012 RO 201200638
(71) Applicant: Institutul de Cercetare, Dezvoltare, Inovare in Stiinte Tehnice si Naturale al Universitatii "aurel Vlaicu" din Arad, 310330 Arad, Jud. Arad (RO)
(72) Inventor: Sirghie, Cecilia, Arad, Jud. Arad (RO); Botar, Alexandru, Cluj Napoca, Jud. Cluj (RO)
(74) Representative: Ghita, Constantin

(57) **Abstract**

The functionalization process of cellulosic textile materials is characterised by the fact that in the first stage it is the cationization of the cellulosic material, in two treatment stages, followed by the actual functionalization stage of the cellulosic material, cationized in two treatment stages. The material's cationization is made in two stages. During the first stage, the treatment of the cellulosic textile material takes place in a strong alkaline environment, with solution of NaOH of HM 1:20, followed by washing, until it reaches neutralisation, and then treatment during the second stage, with cationization agent, 3-chloro-2-hydroxypropyl trimethylammonium chloride of 60% concentration in an alkaline environment, followed by washing, until it reaches neutralisation. The actual functionalization takes place in two stages. During the first stage, there is the reaction between the cationized textile material and the aqueous solution of excess POM, followed by wash of the POM excess. In the second stage, takes place the reaction between POM and Ag and Cu cations, followed by the treatment with the aqueous solution of NaBH₄ in order to obtain Ag and Cu nanoparticles.

## Description

### Field of the invention

This invention refers to a functionalization process of cellulosic textile materials (fibres, threads, fabrics and knitted fabrics), such as: cotton, hemp, flax, ramie, nettle, etc. and different mixes of such fibres. The functionalization process of textile materials develops in two stages, as follows: a first cationization stage, which is performed in two stages, and an actual functionalization stage, which is performed in two stages.

The functionalization of textile materials is a very important process, which provides special properties to products manufactured from them, especially antibacterial and anti-smell properties [T.H.Shemm, S.M.Li, J.H. Wu and C.C. Chen, Textile Res. J., 1993, 63, 357-361., Y.W. Hua, C.J. Chin, H.M. Shan, T.C. Yu, H.P. Hsiung and L.J. Min, J. Appl. Polymer Sciene, 2005, 97, 595-603; P.J. Hauser, C.B. Smith and M.M. Hashem, AUTEX Research Journal, 2004, 4, 1-6; I. Racz and J. Borsa, Textile Res. J., 1998, 68, 69-74; N. Abidi, E. Hequet, S. Tarimala and S. Dai, Process J. Appl. Polym. Sci., 2007, 104, 111-117; R. Rajendran, C. Balakumar, H.A.M. Ahammed, S. Jayakumar, K. Vaideki and E.M. Rejesh, International J. Of Engineering Science and Technology, 2010, 2, 202-208.]

The functionalization of textile materials of natural fibres is an important chemical process because the change of the surfaces plays a decisive part in the modem textile industry [V. Dufaud and F. Lefebvre, Materials, 2010, 18, 682; A. Hebeish, M. Hashem, M. El-Hosamy and S. Abass, RJTA, 2006, 10,73-88; D.T.W. Chan and G.R. Gamble, J. Cotton Sci., 2007, 11,154-158.; N.A. Ibrehim, M. Gouda, A.M. El-Shafel and D.M. Abdel, J. Appl. Polymer Sci., 2007,104, 1754-1761; Y.C. Quin, Y. Zhu, Y. Chen and C. Zhang, J. Appl. Polymer Sci., 2006,101, 766-771; US Patent 6080227/2000; M. Bilgen, P. Hauser and B. Smith, Indian Journal of Textile Research, 2006, 31, 363-368; "Preparation of Ionic Cellulose for Wrinkle Resistant Fabric" P.H. Vaggantwar, Ph.D. Thesis, 2007, Ralaich, North Carolina].

### Backround of the invention

The textile industry developed textile materials with antibacterial properties in order to ensure the staff's sanitary protection against different transmittable diseases. Therefore, many studies have been conducted in order to establish or attach groups of antibacterial substances on textile materials, by using different procedures, out of which:
- Layer sedimentation of metallic nanoparticles (Ag, Au, Pt, Pd, Cu, etc.)
- Graft polymerization of different monomers on cellulosic substances
- Attach quaternary ammonium salts on cotton textiles via covalent bonds.

The disadvantages of the procedures mentioned above refer to the fact that the nanoparticles giving the textile material a certain function (antibacterial or anti-smell), after repeated washing stages (maximum 10 washings) are removed, the material loses the acquired functions and, additionally, metallic particles can be found in the cleaning water, thus leading to environmental pollution.

In order to remove this inconvenient, our research aimed to create an operation procedure that would permanently establish the particles that offer certain functions via electrostatic bonds to the treated materials, particularly silver.

### Solved technical problem

The technical problem solved by this invention is the creation of a composition between biopolymers (cellulose) and inorganic compounds from the class of POM, anions with sizes of 1-2 nanometres, which will be electrostatically connected to the textile materials, which have been previously cationized. Inorganic compounds from the class of POM with Keggin structures that were used in this procedure are negatively charged ions, from -3 to -9, which determine a very strong chemical bond with the textile material, which has been previously cationized. [M.T. Pope, Heteropoly and Isopoly Oxometalates: Springs Verlag; New York, 1983; C.L. Hill and A. Prosser-McCartha, Coord. Chem. Rev., 2000, 143, 407-455]. Additionally, these compounds create ionic bonds with Silver of Copper, thus determining a permanent operation of the textile material, without release of Silver and/or Copper nanoparticles in the waste water resulted from the washing process.

These compounds remain electrostatically fixed, and after repeated washings, they provide the procedure advantages compared to the procedures known so far.

Another technical problem that was solved by this innovation relates to the possibility of obtaining nanoparticles (Silver or Copper) on the textile material, which would offer them numerous properties (for instance, permanent antibacterial and/or antifungal properties).

### Presentation of the invention

The functionalization process of the cellulosic textile materials is characterised by the fact that in the first stage the cationization of the cellulosic material takes place in two treatment stages. The material's cationization is made in two stages. In the first stage it is the treatment of the cellulosic textile material in a strongly alkaline environment with NaOH solution, followed by neutralisation washing, and then treatment in the second stage, with cationization agent, 3-chloro-2-hydroxypropyl trimethylammonium chloride, of 60% concentration in an alkaline environment, followed by neutralisation washing. The actual functionalization takes place in two stages. In the first stage, the reaction between the cationized textile material and the aqueous solution of the exceeding POM takes place, followed by washing the exceeding POM. In the second stage, the reaction between POM and cations of Ag and Cu takes place, followed by treatment with aqueous solution of NaBH_{4,} in order to obtain nanoparticles of Ag and Cu.

According to the invention, the functionalization process of the cellulosic textile materials (fibres, threads, fabrics and knitted fabric) consists of two stages, as follows: a first cationization stage, which consists of two stages, and an actual functionalization stage, which consists of two stages.

The cationization stage of the cellulosic material. First stage: the cellulosic textile material is suspended in aqueous solution containing NaOH. The suspension is slightly shaken up at room temperature for 15-20 hours. After that, the suspension is filtered and washed with water, followed by a wash with diluted solution of acetic acid, and then again with cold water, until it reaches neutral pH.

Cationization stage. The second stage: the material resulted from the first cationization stage is suspended in an aqueous solution containing 0,5%-2,5% NaOH and an addition of 30-50 ml 60% solution 3-chloro-2-hydroxypropyl trimethylammonium chloride. The suspension is well-shaken until it becomes homogenous and it is left to rest for 15-20 hours. The material thus treated is filtered, washed with cold water, then washed with diluted solution of acetic acid and water again, until it reaches neutral pH. The material thus treated is air-dried at room temperature. Functionalization stage with POM compounds. First stage: the cationized cellulosic textile material is first suspended in an aqueous solution 0,2 - 0,4 M POM of type: K₅ H₄ PMo₆ V₆ O_{40,} K₅ H₄ PW₆ V₆ O_{40,} H₅ PMo₁₀ V₂ O_{40,} H₄ SiMO₁₂ O_{40,} it is well-shaken for 10-15 minutes. The material is filtered, washed with water in order to remove exceeding POM and it is air-dried at room temperature. Functionalization stage. The second stage: The textile material cationized and operated with K₅ H₄ PMo₆ V₆ O₄₀ or K₅ H₄ PW₆ V₆ O₄₀ or H₅ PMo₁₀ V₂ O₄₀ or H₄ SiMo₁₂ O₄₀ is suspended in distilled water at a hydromodule of HM 1:15. To this suspension, while shaking it, it is added a solution of 0,1-0,2 N de AgNO₃ or 0,2 -0,4 M (CuSO₄*5H₂O), so that there is a small excess of Ag or Cu ions in the filtrate. The textile material thus treated is left for 5-10 minutes in the AgNO₃ or (CuSO₄*5H₂O) solution, then it is filtered, washed with distilled water until there are no more Ag or Cu ions left. In order to obtain Ag or Cu nanoparticles, another treatment stage is added on the textile material, as follows: the material operated with Ag or Cu salts is suspended in distilled water, in an HM 1:20., , A cold aqueous solution containing 0,2% weak reducer agent is added to this suspension while shaking it. After 15-20 minutes, the textile material is filtered and washed with distilled water. The textile material is air-dried, at room temperature.

### Advantages

Materials treated following the procedure described by this invention keep their properties even after 10 repeated washings, nanoparticles remain attached to the material, and they do not get discharged into cleaning waters, thus contributing to environmental protection.

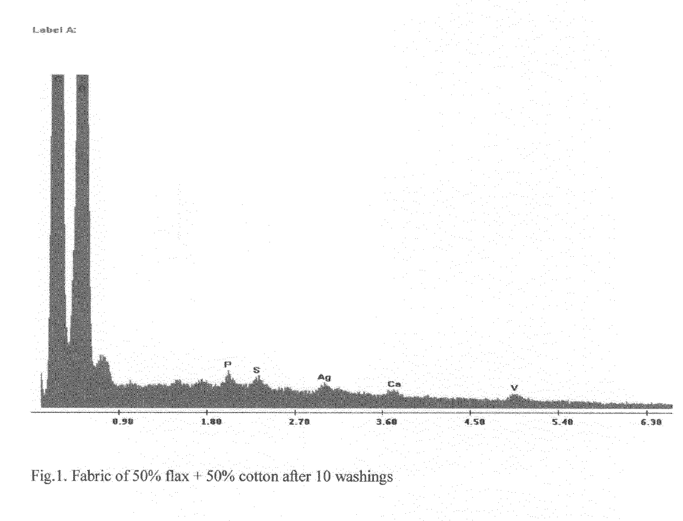

In order to prove these advantages, in fig. 1 it is presented the EDAX analysis on the fabrics operated and washed 10 times. From the figure it results that on the operated textile material there are both P and V components of the corresponding POM, namely Ag nanoparticles.

### Example

An example of invention application can be found below:
Cationization stage of the cellulosic material. First stage:
   a) 20 g of cellulosic textile material are suspended in 200 ml aqueous solution, which contains NaOH. The suspension is slightly shaken at room temperature for 20 hours. Then, the suspension is filtered and washed, firstly with water, and then with a solution of 2% acetic acid, and then again with cold water, until it reaches neutral pH.
Cationization stage. Second stage:
   b) 20 g of the material resulted in the first cationization stage is suspended in 400 ml aqueous solution containing 2,3 g NaOH and 50 ml 60 % solution 3-chloro-2-hydroxypropyl trimethylammonium chloride. The suspension is well-shaken until it becomes homogenous and it is left to rest for 14-20 hours. The material thus treated is filtered, firstly washed with cold water, and then with a solution of 2% acetic acid, and then again water, until it reaches neutral pH. The material thus treated is air-dried at room temperature.
Functionalization stage with POM compounds. First stage.
   c) 100 g of cationized cellulosic textile material is firstly suspended in one litre aqueous solution 0,2 M POM of type: K₅ H₄ PMo₆ V₆ O_{40,} K₅ H₄ PW₆ V₆ O_{40,} H₅ PMo₁₀ V₂ O_{40,} H₄ SiMo₁₂ O_{40,} it is shaken for 10-15 minutes. It can be observed that the material will colour, which shows that the reaction with the POM is immediate. The material is filtered, washed with water in order to remove the excess POM and it is air-dried at room temperature. In order to validate the ionic bond between POM and the cationized textile material, the following test will be taken: samples of 5 g of operated material, in the second stage, will be suspended in 250 ml of distilled water, shaken until it becomes homogenous and left to rest for a month at room temperature, while constantly shaking it. After filtration, there will be no trace of POM in the filtrate via UV-VIS spectra, as it will remain attached by ionic bond to the textile material. FTIR spectra created on the operated material confirm the presence of POM on the material.
Functionalization stage. Second stage:
   d) 50 g of material cationized and operated with K₅ H₄ PMo₆ V₆ O₄₀ or K₅ H₄ PW₆ V₆ O₄₀ or H₅ PMo₁₀ V₂ O₄₀ or H₄ SiMo₁₂ O_{40,} is suspended in 250 ml distilled water. To this suspension, it is added, while shaking it, and in small quantities of 1-2 ml, a solution of 0,1 N de AgNO₃ or 0,2 M (CuSO₄*5H₂O), so that there is a small excess of Ag or Cu ions in the filtrate. The textile material thus treated is left for 5-10 minutes in the AgNO₃ or (CuSO₄*5H₂O) solution, then it is filtered, washed with distilled water until there are no more Ag or Cu ions. If it is wished to achieve Ag or Cu nanoparticles on the textile material, then it is applied an additional treatment stage, as follows: 50 g of the material operated with Ag or Cu salts is suspended in 400 ml of distilled water. To this suspension, it is added, while shaking it, a cold aqueous solution containing 0,2 g NaBH₄ in 100 ml water. After 15-20 minutes, the textile material is filtered and washed with distilled water. The material is air-dried at room temperature.

## Claims

1. The procedure of functionalization of textile materials (fibers, yarns, woven and knitted fabrics), cellulose materials (cotton, hemp, flax, ramie, nettles and different mixtures of these fibers, such as: flax-cotton, hemp-cotton, etc.), according to the invention is **characterized by** the fact that it is being accomplished in two phases, in the first phase takes place the cationization of the cellulose material by the suspension into a water solution that contains10-20% NaOH with a liquid ratio fabric to solution of 1:10, for 15-20 hours, then washing with water and diluted solution of acetic acid until the neutral pH is obtained, and then follows the suspension of the material for 15-20 hours in a water solution that contains 0,5%-2,5% NaOH in HM 1:80 and an addition of 30-50 ml of solution 60% of 3-chloro 2-hydroxypropyl trimethylammonium, followed by repeated washes with cold water and diluted solution of acetic acid until the neutral pH is obtained, drying at the room's temperature, and in the second phase takes place the functionalization of the textile cellulose material by its immersion for 10-15 minutes, stirring in a water solution 0,2 - 0,4 M polyoxometalates of type: K₅ H₄ PMo₆ V₆ O_{40,} K₅ H₄ PW₆ V₆ O_{40,} H₅ PMo₁₀ V₂ O_{40,} H₄ SiMo₁₂ O₄₀, after which it is being filtered and suspended for 5-10 min in 200 - 500 ml distilled water to which it is being added while stirring a solution of 0,1-0,2 N of AgNO₃ or 0,2 - 0,4 M (CuSO₄*5H₂O), followed by washing for 15-20 minutes with a solution of 0,2% NaBH₄ with a liquid ratio fabric to solution of 1:20. In the end the material is washed with distilled water and it is dried in the air at the room's temperature.

2. The functionalization procedure according to claim 1, assures the textile material with antibacterial and antifungal properties through the silver and copper nanoparticles fixed on the fabric.

3. The functionalization procedure according to claim 1 is **characterized by** the fact that the antibacterial and antifungal properties are guaranteed even after 10 washes.
